# EUROPEAN PATENT SPECIFICATION

(11) **EP 4 125 885 B1**
(45) Date of publication and mention of the grant of the patent: **25.12.2024**
(21) Application number: 21713990.6
(22) Date of filing: 26.03.2021
(51) Int. Cl.: A61K 31/4365, A61P 3/10, A61P 13/12

(54) **USE OF A THIENOPYRIDONE DERIVATIVE IN THE TREATMENT OF DIABETIC NEPHROPATHY**
VERWENDUNG EINES THIENOPYRIDONDERIVATS ZUR BEHANDLUNG VON DIABETISCHER NEPHROPATHIE
UTILISATION D'UN DÉRIVÉ DE THIOPYRIDONE DANS LE TRAITEMENT D'UNE NÉPHROPATHIE DIABÉTIQUE

(30) Priority: 30.03.2020 EP 20166749
(43) Date of publication of application: 08.02.2023
(73) Proprietor: Poxel, 69007 Lyon (FR)
(72) Inventor: BOLZE, Sébastien, 01600 MASSIEUX (FR); FOUQUERAY, Pascale, 5643 SINS (CH); HALLAKOU-BOZEC, Sophie, 92160 ANTONY (FR)
(74) Representative: Cabinet Becker et Associés
(86) International application number: PCT/EP2021/058005
(87) International publication number: WO 2021/198099

(56) References cited:
- WO-A1-2014/001554
- SALATTO CHRISTOPHER T. ET AL: "Selective Activation of AMPK [beta] 1-Containing Isoforms Improves Kidney Function in a Rat Model of Diabetic Nephropathy", JOURNAL OF PHARMACOLOGY AND EXPERIMENTAL THERAPEUTICS, vol. 361, no. 2, 1 May 2017 (2017-05-01), US, pages 303 - 311, XP055809203, ISSN: 0022-3565, Retrieved from the Internet <URL:https://jpet.aspetjournals.org/content/jpet/361/2/303.full.pdf> DOI: 10.1124/jpet.116.237925
- K. MCMAHON ET AL: "The Role of 5-AMP-Activated Protein Kinase (AMPK) in Diabetic Nephropathy: A New Direction?", CURRENT ENZYME INHIBITION, vol. 5, no. 1, 1 February 2009 (2009-02-01), pages 44 - 50, XP055154069, ISSN: 1573-4080, DOI: 10.2174/157340809787314256
- STEINBERG GREGORY R ET AL: "AMP-activated protein kinase: the current landscape for drug development", NATURE REVIEWS DRUG DISCOVERY, NATURE PUBLISHING GROUP, GB, vol. 18, no. 7, 13 March 2019 (2019-03-13), pages 527 - 551, XP037134897, ISSN: 1474-1776, [retrieved on 20190313], DOI: 10.1038/S41573-019-0019-2

## Description

### TECHNICAL FIELD

The invention relates to the use of a thienopyridone derivative in the treatment of diabetic nephropathy.

### TECHNICAL BACKGROUND

Diabetic nephropathy is a renal disorder having a complex etiology because of an interplay between systemic metabolic and vascular dysfunctions. It is caused, *inter alia,* by hyperglycemia and hyperlipidemia, which are associated with diabetes. This condition manifests in inflammation and fibrosis leading to the impairment of both glomerular filtration and tubular function.

Current therapies such as antidiabetic therapies that lower glucose reduce progression but do not halt disease progression to end-stage renal disease which requires dialysis or transplant. Although there is no current treatment of diabetic nephropathy, a number of compounds have been proposed or even tested in clinical trials, which includes AMPK activators.

AMPK is indeed known to control key regulatory steps in lipid biosynthesis and lipid oxidation as well as regulation of glucose homeostasis. AMPK has 12 possible heterotrimers which consist of an α (α1, α2) catalytic subunit, a regulatory and structurally crucial β (β1, β2) subunit, and a regulatory γ (γ1, γ2, γ3) subunit. These isoforms are encoded for by distinct genes that are differentially expressed and have unique tissue specific expression profiles. Among them, the subunit β1 appears to be the predominant subunit in kidney. To improve kidney function, it has thus been suggested use indirect and direct AMPK activators, such as metformin, AICAR and a number of other small molecules which selectively activate AMPK heterotrimers containing the β1 subunit, such as Compound 991 from Merck, PF-249 from Pfizer (C.T. Salatto et al., J. Pharmacol. Exp. Ther., 361(2), 303-311, 2017) and A-799662 from Abbott, which is a thienopyridone with the following structure:

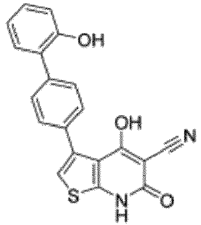

However, A-799662 can only be administered by injection due to poor oral bioavailability, which represents a strong limitation that may have precluded its development.

An indole acid (PF-06409577) which is a β1 selective direct AMPK activator showed efficacy in a preclinical model of diabetic nephropathy (K.O. Cameron et al., J. Med. Chem., 59, 17, 8068-8081, 2016). Although PF-06409577 entered a phase I clinical trial for the treatment of diabetic nephropathy, this study was not completed. More recently, it has been suggested than another direct activator of AMPK, i.e. MK-8722 from Merck, could not only show a beneficial preventative effect but also modulate established renal disease (X. Zhou et al., J. Pharmacol. Exp. Ther., 373(1), 45-55, 2020). However, this compound is also known to trigger cardiac side effects.

However, there remains the need for alternative compounds that could be administered orally in the treatment of diabetic nephropathy at clinically relevant doses and/or with reduced side effects.

The inventors have now shown that specific thienopyridone derivatives could satisfy this need. These compounds are direct activators of AMPK isoforms including the β1 subunit, which are encompassed within the generic formula of AMPK activators disclosed in WO 2014/001554 but it has never been suggested so far to use them in the treatment of diabetic nephropathy. In addition, they display a higher potency on AMPK activation, a highest efficacy on glycemic and lipidic metabolism and a highest oral bioavailability than other compounds disclosed in WO 2014/001554.

### SUMMARY OF THE INVENTION

Specifically, this invention relates to a thienopyridone derivative of Formula (I): 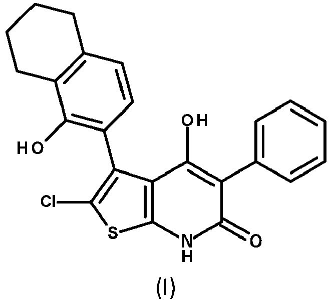
or its pharmaceutically acceptable salts and/or solvates,
or a pharmaceutical composition comprising the same,
for use in the treatment of diabetic nephropathy.

The present invention also relates to a pharmaceutical composition comprising an effective amount of a thienopyridone derivative as described above and a pharmaceutically acceptable support.

The present invention also relates to the use of a thienopyridone derivative as described above, or a pharmaceutical composition comprising the same, for the manufacture of a medicament for the treatment of diabetic nephropathy.

The invention is defined in the appended claims.

### BRIEF DESCRIPTION OF THE FIGURES

**Figure 1** shows the glomerular filtration rate observed in ZSF1 rats treated with PXL770 compared to untreated ZSF1 rats and to Lean rats.
**Figure 2** shows the urine albumin level (albuminuria) observed in ZSF1 rats treated with PXL770 compared to untreated ZSF1 rats and to Lean rats.
**Figure 3** shows the urinary volume and glycosuria observed in ZSF1 rats treated with PXL770 compared to untreated ZSF1 rats and to Lean rats.

### DETAILED DESCRIPTION OF THE INVENTION

This invention pertains to specific uses of thienopyridone derivatives of Formula (I): 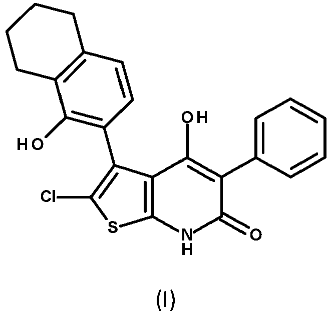 and their pharmaceutically acceptable salts and/or solvates.

The compound of formula (I) and a preparation process thereof have been disclosed in patent application WO 2014/001554.

Alternatively, said compound of formula (I) may be obtained by an improved process comprising the steps of:
(a) reacting 6-acetyl-5-hydroxytetralin with an electrophilic benzyl source, preferably benzyl bromide, in the presence of a base;
(b) reacting the compound obtained in step (a) with ethyl cyanoacetate in the presence of hexamethyldisilazane and acetic acid;
(c) reacting the compound obtained in step (b) with sulfur in the presence of a base;
(d) optionally forming a salt of the compound obtained in step (c), preferably a hydrochloride salt;
(e) reacting the compound obtained in step (c) or (d) with an electrophilic chlorine source, preferably N-chlorosuccinimide;
(f) reacting the compound obtained in step (e) with phenylacetyl chloride;
(g) reacting the compound obtained in step (f) with a base;
(h) reacting the compound obtained in step (g) with boron tribromide or trichloride, preferably boron trichloride; and
(i) optionally recovering the compound obtained in step (h).

Typically, step (B) can comprise a substep (b1) of heating the mixture obtained in step (A), preferably at a temperature close to reflux of the mixture, followed by a substep (b2) of cooling the resulting mixture, for instance at a temperature comprised between -15 °C and 35 °C. The expression "close to reflux of the mixture" refers typically to a temperature comprised between 90% and 100 % of the boiling point of the solvent system in step (A) (for instance, water/isopropanol or water/*n*-butyl acetate).

A distillation step, preferably under reduced pressure, can be carried out between the heating substep and substep (b2).

Step (B) allows a crystalline precipitate to form, which formation may be favored or triggered by adding seeds to steps (b2).

In a preferred embodiment, said precipitate is recovered by filtration in step (C). It may then be washed successively with one or more solvents, preferably water, *n*-butyl acetate and/or *tert*-butyl methyl ether.

Examples of pharmaceutically acceptable salts of the compound of formula (I) can be obtained by reacting the compound of formula (I) with various organic and inorganic bases by procedures usually known in the art to give the corresponding base-addition salt. Such bases are, for example, alkali metal hydroxides, including potassium hydroxide, sodium hydroxide and lithium hydroxide; alkali metal carbonates, including potassium carbonate and sodium carbonate; alkaline earth metal hydroxides, such as barium hydroxide and calcium hydroxide; alkaline earth metal carbonates; alkali metal alkoxides, for example potassium ethoxide and sodium propoxide; and various organic bases, such as piperidine, diethanolamine and N-methylglutamine. The aluminium salts of the compounds of formula (1) are likewise included.

The salts of the compound of formula (I) thus include aluminium, ammonium, calcium, copper, iron(III), iron(II), lithium, magnesium, manganese(III), manganese(II), potassium, sodium and zinc salts, but this is not intended to represent a restriction. Of the above-mentioned salts, preference is given to the mono-, di- and tri- sodium or potassium salts and most preferably to the potassium salts.

Any of the pharmaceutically acceptable salts of the compound of formula (I), or this compound itself, may be used in this invention in the form of one of its solvates. "Solvates" of the compounds are taken in the present invention to mean adductions of inert solvent molecules onto the compounds which form owing to their mutual attractive force. The nature of the solvate thus depends on the solvent used during the reaction of the base with the compound of formula (I). Examples of solvates include alcohol solvates, for instance methanol or ethanol solvates, and hydrates, including mono-, di-, tri- or tetrahydrates, but this is not intended to represent a restriction.

In a most preferred embodiment of this invention, the compound used in this invention is the monohydrate potassium salt of the compound of formula (I), corresponding to the following structure of formula (Ia):

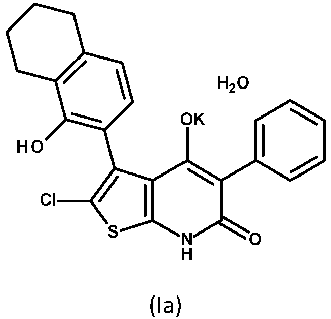

This compound may be prepared according to a process comprising the steps of:
(A) reacting the compound of formula (I) with potassium carbonate in a solution comprising water and a solvent selected from n-butyl acetate and isopropanol:
(B) forming a precipitate; and
(C) recovering the precipitate obtained in step (B), preferably by filtration.

This process allows obtaining the monohydrate potassium salt of 2-chloro-4-hydroxy-3-(5-hydroxytetralin-6-yl)-5-phenyl-7H-thieno[2,3-b]pyridin-6-one, also known as PXL770, which is obtained in the form of a solid, such as a powder, having the following XRPD (X-Ray Powder Diffraction) peaks, as measured by means of a diffractometer, using Cu K(alpha) radiation:

| 2-theta (°) | d-value (Å) |
|---|---|
| 13.010 | 6.7992 |
| 14.720 | 6.0130 |
| 17.330 | 5.1128 |
| 19.640 | 4.5164 |
| 21.170 | 4.1933 |
| 22.700 | 3.9140 |
| 23.860 | 3.7263 |
| 24.410 | 3.6435 |
| 26.730 | 3.3323 |
| 28.700 | 3.1079 |
| 30.960 | 2.8860 |
| 34.750 | 2.5794 |
| 35.530 | 2.5246 |
| 35.950 | 2.4960 |
| 36.660 | 2.4493 |

In the following description, "the thienopyridone derivative" is intended to mean both the base compound of formula (I), its solvates, its salts and the solvates of its salts.

In the present invention, the thienopyridone derivative or a pharmaceutical composition comprising the same are used in the treatment of diabetic nephropathy.

The pharmaceutical composition used according to the invention may be prepared by any conventional method. The thienopyridone derivative can be converted into a suitable dosage form together with at least one solid, liquid and/or semi-liquid excipient or adjuvant and, if desired, in combination with one or more further active ingredients.

The term "pharmaceutically acceptable support" refers to carrier, adjuvant, or excipient acceptable to the subject from a pharmacological/toxicological point of view and to the manufacturing pharmaceutical chemist from a physical/chemical point of view regarding to composition, formulation, stability, subject acceptance and bioavailability.

The term "carrier", "adjuvant", or "excipient" refers to any substance, not itself a therapeutic agent, that is added to a pharmaceutical composition to be used as a carrier, adjuvant, and/or diluent for the delivery of a therapeutic agent to a subject in order to improve its handling or storage properties or to enable or facilitate formation of a dosage unit of the composition into a discrete article. The pharmaceutical compositions of the invention, either individually or in combination, can comprise one or several agents or vehicles chosen among dispersants, solubilisers, stabilisers, preservatives, etc.

The terms "treatment", "treating" and "treat" refer to therapy, prevention and prophylaxis of diabetic nephropathy or at least one of its symptoms. This also means an improvement, prevention of at least one measurable physical parameter associated with the disease being treated, which is discernible or not in the subject. The term "treatment" or "treating" further refers to inhibiting or slowing the progression of the disease, physically, stabilization of a discernible symptom, physiologically, for example, stabilization of a physical parameter, or both. The term "treatment" or "treating" also refers to delaying the onset of the disease. In some particular embodiments, the compound of the invention is administered as a preventive measure. In this context, "prevention" or "preventing" refers to a reduction in the risk of developing at least one of the symptoms related to the disease.

The term "treating " can thus include preventing an increase in, or decreasing proteinuria, especially albuminuria, preventing or reducing kidney fibrosis, preventing the decrease or increasing glomerular filtration rate (GFR), reducing or preventing the increase in urine glucose level, reducing or preventing the increase in urine volume by the subject, and combinations thereof, with the thienopyridone derivative or a pharmaceutical composition comprising the same.

The treatment involves the administration of the thienopyridone derivative or a pharmaceutical composition of the invention to a subject having declared diabetic nephropathy to cure, delay, or slow down the progress, thus improving the condition of patients.

Within the context of the invention, the term "subject" means a mammal and more particularly a human. The subjects to be treated according to the invention can be appropriately selected on the basis of several criteria associated with the disease.

Pharmaceutical compositions can be administered in the form of dosage units which comprise a predetermined effective amount of active ingredient per dosage unit.

Pharmaceutical compositions can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such compositions can be prepared using all processes known in the pharmaceutical art by, for example, combining the active ingredient with the excipient(s) or adjuvant(s). Preferably, the pharmaceutical composition according to the invention is adapted for oral administration.

Pharmaceutical compositions adapted for oral administration can be administered as separate units, such as, for example, capsules or tablets; powders or granules; solutions or suspensions in aqueous or non-aqueous liquids; edible foams or foam foods; or emulsions, such as oil-in-water liquid emulsions or water-in-oil liquid emulsions.

Thus, for example, in the case of oral administration in the form of a tablet or capsule, the active ingredient component can be combined with an oral, non-toxic and pharmaceutically acceptable inert excipient. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules may be produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes, and the like. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum and the like. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbent, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tableting machine, giving lumps of non-uniform shape which are broken up to form granules. The granules can be lubricated by addition of stearic acid, a stearate salt, talc or mineral oil in order to prevent sticking to the tablet casting moulds. The lubricated mixture is then pressed to give tablets. The compound according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Pharmaceutical compositions adapted for oral administration can also be formulated by spray drying of a solid or liquid dispersion.

Oral liquids, such as, for example, solution, syrups and elixirs, can be prepared in the form of dosage units so that a given quantity comprises a prespecified amount of the compound. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners and the like, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax and the like.

The thienopyridone derivative used according to the invention can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

By "effective amount" it is meant the quantity of the compound as defined above which prevents, removes or reduces the deleterious effects of the treated disease in humans. It is understood that the administered dose may be adapted by those skilled in the art according to the patient, the pathology, the mode of administration, etc. For instance, the thienopyridone derivative may be administered once or twice a day at a daily dose of 0.5mg to 300 mg for a human patient, preferably from 20 mg to 1000 mg, more preferably from 60 mg to 500 mg. It can be administered 4, 5, 6 or 7 days a week as a long-life medication. In a particular embodiment of this invention, the thienopyridone derivative is administered as dosage units which comprise from 0.5 mg to 1500 mg, preferably from 20 mg to 1000 mg, more preferably from 60 mg to 500 mg of the thienopyridone derivative.

The invention will also be described in further detail in the following examples, which are not intended to limit the scope of this invention, as defined by the attached claims.

### EXAMPLES

### Example 1: Synthesis of PXL770

### Analytical methods

### XRPD

X-Ray Powder Diffraction (XRPD) analyses were performed using a Panalytical Xpert Pro diffractometer equipped with a Cu (K alpha radiation) X-ray tube and a Pixcel detector system. The samples were analysed in transmission mode and held between low density polyethylene films. XRPD patterns were sorted, manipulated and indexed using HighScore Plus 2.2c software.

### TG/DTA

Thermogravimetric (TG) analyses were carried out on a Perkin Elmer Diamond Thermogravimetric/Differential Temperature Analyser (TG/DTA). The calibration standards were indium and tin. Samples were placed in an aluminium sample pan, inserted into the TG furnace and accurately weighed. The samples were heated from 30-300°C in a stream of nitrogen at a rate of 10°C/minute. The temperature of the furnace was equilibrated at 30°C prior to the analysis of the samples.

### 10) Synthesis of 1-(5-benzyloxytetralin-6-yl)ethanone (1)

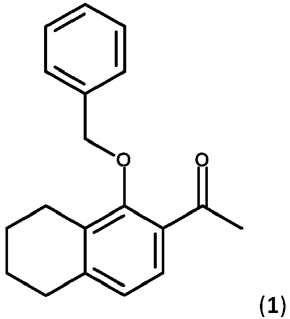

6-Acetyl-5-hydroxytetralin (100 g, 1 eq.) was dissolved in acetonitrile (300 mL). After addition of K₂CO₃ (1.1 eq.) and benzyl bromide (1.05 eq.), the suspension was heated (76°C). After 48 hours, benzyl bromide (0.1 eq) was added. After overall 74 hours, the solid was filtered off and washed with acetonitrile (200 mL), and the combined filtrates were evaporated.

Compound **1** was obtained as a syrup: m = 148.6 g, quantitative yield, 96.6% a/a purity.

### 1b) Synthesis of ethyl 2-amino-4-(5-benzyloxytetralin-6-yl)thiophene-3-carboxylate (2)

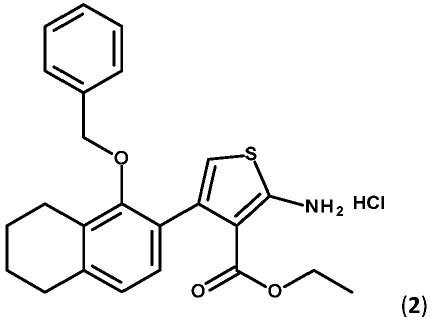

Acetic acid (70 mL) was heated to T = 65°C. HMDS (1.5 eq.) was added over 10 min. Afterwards, a solution of compound 1 (69.5 g, 1 eq.) and ethyl cyanoacetate (1.5 eq.) in acetic acid (140 mL) was added. The resulting mixture was stirred at T = 65°C for 24 h.

After cooling to room temperature, aqueous NaOH (1 M, 140 mL) and TBME (210 mL) were added. The layers were separated. The organic layer was washed with aqueous NaOH (1 M, 4 × 140 mL) until the pH of the aqueous phase was basic (pH = 13). The organic layer was washed with aqueous HCl (1M, 140 mL) and H₂O (2 × 140 mL).

EtOH (240 mL), NaHCO₃ (1.3 eq.) and sulfur (1.0 atom eq.) were added. After heating to reflux for 180 min, the reaction mixture was concentrated to 210 mL and co-evaporated with TBME (3 × 140 mL). After cooling to room temperature, the suspension was filtered and the solid was washed with TBME (70 mL). The combined filtrates were concentrated to 210 mL and HCl in dioxane (1.1 eq.) was added dropwise at room temperature. After seeding, precipitation was observed. Heptane (350 mL) was added dropwise at room temperature. After stirring for 14 h, the suspension was filtered. After washing with heptane (3 × 70 mL) and drying, compound **2** was recovered as a solid. m = 83.2 g, 71% yield, 93.7% a/a purity.

### 1c) Synthesis of ethyl 4-(5-benzyloxytetralin-6-yl)-5-chloro-2-((2-phenylacetyl)amino] thiophene-3-carboxylate (3)

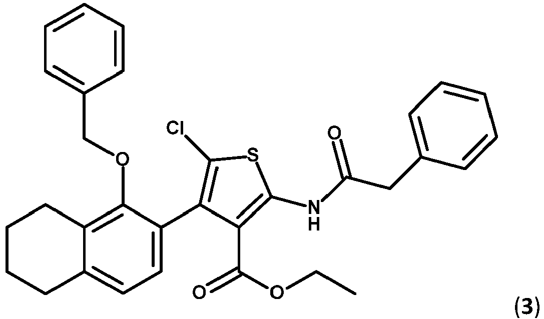

Compound **2** (17.69 g, 1 eq.) was dissolved in dichloromethane (140 mL). The resulting solution was cooled with ice/water. Under stirring, N-chlorosuccinimide (1.05 eq.) was added. The mixture became dark over a few minutes. After 1 h, phenylacetyl chloride (1.25 eq.) was added.

After 1 hour at 0 °C and 2 hours at room temperature, the mixture was evaporated down to ca. 35 mL and EtOH (2 × 70 mL) was added, and evaporated down again. The mixture was diluted with EtOH (35 mL) and cooled with ice/water. The product precipitated. The solid was filtrated and washed with cold EtOH (3 × 18 mL).

Compound **3** was obtained as a solid: m = 20.99 g, 94.2 % yield, 99.3 % a/a purity.

### 1d) Synthesis of 3-(5-benzyloxytetralin-6-yl)-2-chloro-4-hydroxy-5-phenyl-7H-thieno[2,3-b]pyridin-6-one (4)

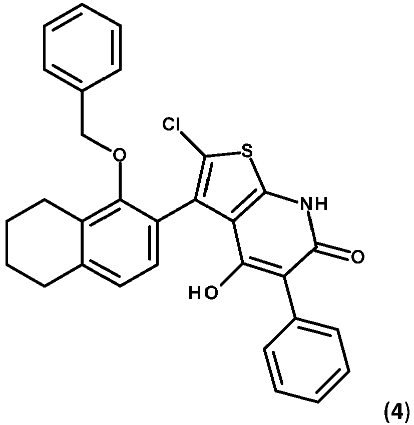

Compound **3** (19.88 g, 1 eq.) was solubilized in methyltetrahydrofuran (120 mL), and the reaction mixture was cooled to a temperature between -16°C and -10 °C (NaCl/Ice). Potassium tert-butoxide (5 eq.) was added in four portions. Then, the reaction mixture was warmed up to room temperature, and stirred for 65 min at room temperature. A dropwise addition of 2N HCl (5 eq.) was carried out at T = 0-5°C (water/ice) and the resulting mixture was stirred vigorously. The organic phase was washed with NaCl_{(aq)} (11%, 1 × 50 mL) and water (2 × 50 mL). The organic phase was concentrated to ~50% solution. Methyltetrahydrofuran (80 mL) was added, and the resulting solution was concentrated to ~50% solution. TBME (100 mL) was added, and the resulting solution was concentrated to ~50% solution (this step was repeated 3 times). Then, TBME (25 mL), seeds of compound **4** and n-Heptane (20 mL) were added and the resulting solution was stirred at room temperature overnight. The mixture was concentrated to ca. 50 mL, filtrated, rinsed with mother liquor and washed with n-Heptane (2 × 40 mL) and dried. Compound **4** was obtained as a granular solid. Yield 88 %, 99.5 % a/a purity.

### 1e) Synthesis of 2-chloro-4-hydroxy-3-(5-hydroxytetralin-6-yl)-5-phenyl-7H-thieno[2,3-b]pyridin-6-one (I)

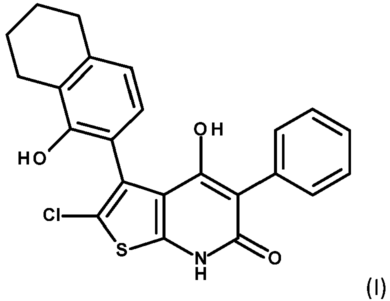

Compound 4 (15 g, 1eq.) was dissolved in 75 mL of dichloromethane and was cooled to T = -10°C/-15°C (with ice/NaCl). BCl₃ (1.5 eq., solution: 1 mol/L in dichloromethane) was added dropwise and the resulting mixture was stirred at room temperature for 15 hours. The resulting mixture was cooled with ice/water, and water (75 mL) was added. The resulting mixture was stirred vigorously and the organic phase was extracted with water/MeOH (9:1 v/v, 5 × 45 mL.). The organic phase was concentrated, a solvent swap was carried out with toluene (3 × 90 mL) and diluted with toluene to reach a final volume of 90 mL of toluene. The resulting mixture was heated to reflux and 15 mL of methanol was added. A brownish solution with few particles was obtained. Seeds were added at T = 40 °C, warmed to T = 52°C and cooled to room temperature. The resulting mixture was stirred overnight, and then was cooled with ice/NaCl (T = -10°C/-15°C) for 100 minutes. The precipitated product was filtrated, washed with toluene/heptane 1:2 v/v (15 mL) and heptane (15 mL) and dried. Crystals of compound (I) were obtained: 87 % yield, 99.0 % a/a purity.

### 1f) Synthesis of the monohydrate potassium salt of 2-chloro-4-hydroxy-3-(5-hydroxytetralin-6-yl)-5-phenyl-7H-thieno[2,3-b]pyridin-6-one (Ia)

Compound (I) was suspended in water/isopropanol mix (1/1, 5 parts of each solvents) then 0.50 to 0.55 eq of potassium carbonate was added. The pH was about 12 (pH indicator paper) at the end of the addition of potassium carbonate. After 3 hours of stirring at 50°C, the suspension was thicker and the pH was about 8 (pH indicator paper). The temperature was raised to 80 °C until a solution was obtained (10-15 minutes). A clarification can be done at this point of the process if required. 7 parts of water were added and the reaction mixture was then cooled to 40°C (turbid solution observed). The solvent was distilled under reduce pressure (from 180mbar to 40mbar) at 40°C until 7 parts of solvents remained in the reactor. Crystallization of potassium salt monohydrate may occur here. 4.2 parts of water were added and the mixture was seeded with compound (I) (1 to 2% of seeds). The suspension was then cooled down from 40°C to 5°C in 7 hours (5°C/hour) and kept at 5°C for several hours. The suspension was filtered. The cake was washed twice by 1.42 parts of water. The collected solid was dried at 40°C under vacuum given minimum 80% yield of Compound (Ia), at required chemical purity (i.e. 98%+).

### Example 2: Characterization of PXL770

a) X-ray powder diffraction (XRPD) data of compound (Ia) indicated that it was composed of a crystalline material. The XRPD description of compound (Ia) is shown in Table 1.

**Table 1**

| Peak No | 2-theta (°) | d-value (Å) | Relative intensity (%) |
|---|---|---|---|
| 1 | 4.910 | 17.9826 | 15 |
| 2 | 11.560 | 7.6486 | 8 |
| 3 | 13.010 | 6.7992 | 25 |
| 4 | 14.720 | 6.0130 | 100 |
| 5 | 16.450 | 5.3843 | 11 |
| 6 | 17.330 | 5.1128 | 49 |
| 7 | 17.770 | 4.9872 | 14 |
| 8 | 18.690 | 4.7437 | 12 |
| 9 | 19.220 | 4.6141 | 16 |
| 10 | 19.640 | 4.5164 | 20 |
| 11 | 20.190 | 4.3946 | 8 |
| 12 | 21.170 | 4.1933 | 23 |
| 13 | 21.580 | 4.1145 | 12 |
| 14 | 22.190 | 4.0028 | 12 |
| 15 | 22.700 | 3.9140 | 26 |
| 16 | 23.240 | 3.8243 | 17 |
| 17 | 23.860 | 3.7263 | 23 |
| 18 | 24.410 | 3.6435 | 43 |
| 19 | 25.330 | 3.5133 | 10 |
| 20 | 26.230 | 3.3947 | 17 |
| 21 | 26.730 | 3.3323 | 23 |
| 22 | 28.700 | 3.1079 | 25 |
| 23 | 29.590 | 3.0164 | 11 |
| 24 | 29.950 | 2.9810 | 13 |
| 25 | 30.960 | 2.8860 | 36 |
| 26 | 31.570 | 2.8316 | 15 |
| 27 | 32.200 | 2.7776 | 18 |
| 28 | 33.080 | 2.7057 | 14 |
| 29 | 33.530 | 2.6704 | 17 |
| 30 | 34.050 | 2.6308 | 10 |
| 31 | 34.750 | 2.5794 | 26 |
| 32 | 35.530 | 2.5246 | 56 |
| 33 | 35.950 | 2.4960 | 22 |
| 34 | 36.660 | 2.4493 | 20 |
| 35 | 37.300 | 2.4087 | 11 |
| 36 | 38.320 | 2.3469 | 16 |
| 37 | 39.490 | 2.2801 | 13 |

b) TG/DTA analysis showed an initial weight loss of 1.1% from 30-100°C, followed by larger weight loss of 3% from 117-160°C due to loss of bound water. The second weight loss was accompanied by a large endotherm and the combined weight losses of 4% approximate the theoretical weight loss for a monohydrate (3.75% w/w). The compound decomposed above 240°C.

### Example 3: In vivo experiments

### Introduction

ZSF1 rat is a Leptin-resistant, obese, hypertensive Zucker diabetic fatty/Spontaneously hypertensive heart failure F1 hybrid (ZSF1) rat. The obese ZSF1 rat is described as a relevant pre-clinical model for the study of renal function decline to end stage renal disease in the setting of type 2 diabetes (Dower K et al., PLoS ONE 2017; 12(7): e0181861). It exhibits many of the traits of human diabetic nephropathy (Boustany-Kari C et al., J Pharmacol Exp Ther 2016; 356:712-719).

### Methods and Materials

Obese ZSF1 rats and lean ZSF1 (Lean) were 12-week-old at the time of treatment initiation. The following groups were constituted:
- Lean rats (untreated; n=5-8)
- ZSF1 rats (untreated; n=9-12)
- ZSF1 treated with PXL770 (n=8-12)

The compound of Example 2, also known as PXL770 was administered by oral gavage at the dose of 150 mg/kg twice a day for 8 days (13 weeks of age) or 90 days (24-week old of age). The PXL770 suspension was prepared at the concentrations used in the study (150 mg/kg) in the vehicle, i.e. carboxymethylcellulose 0.5% / Tween 80 (98/2).

### Investigated Parameters - Renal function

### Glomerular filtration rate (GFR)

At D₈ and D₉₀, glomerular filtration rate was determined with the fluorescent glomerular filtration rate tracer fluorescein isothiocyanate (FITC)-sinistrin using the transcutaneous glomerular filtration rate system (NIC-Kidney; Mannheim Pharma and Diagnostics GmbH, Mannheim, Germany) (Schock-Kusch D. et al., PLoS One 2013;8, e71519).

### Albuminuria, glycosuria and urine volume

Randomly selected animals of each group (n=8-12) were placed in separate metabolic cages in order to collect 15h over-night urine samples at D₈ and D₉₀. Albuminuria was determined as indicator of renal dysfunction using IDEXX Vetlab analyzer. Glycosuria was determined using IDEXX Vetlab analyzer.

### Statistical Analysis

All results will be given as mean ± SEM.

In order to evaluate the effect of the pathology, all parameters obtained in untreated obese ZSF1 and Lean rats were compared by Student's unpaired two-tailed t-test.

In order to evaluate the effects of PXL770, all parameters obtained in 8- and 90-day PXL770-treated ZSF1 rats were compared with age-matched untreated ZSF1 rats using Student's unpaired two-tailed t-test.

### Results

### Glomerular filtration rate

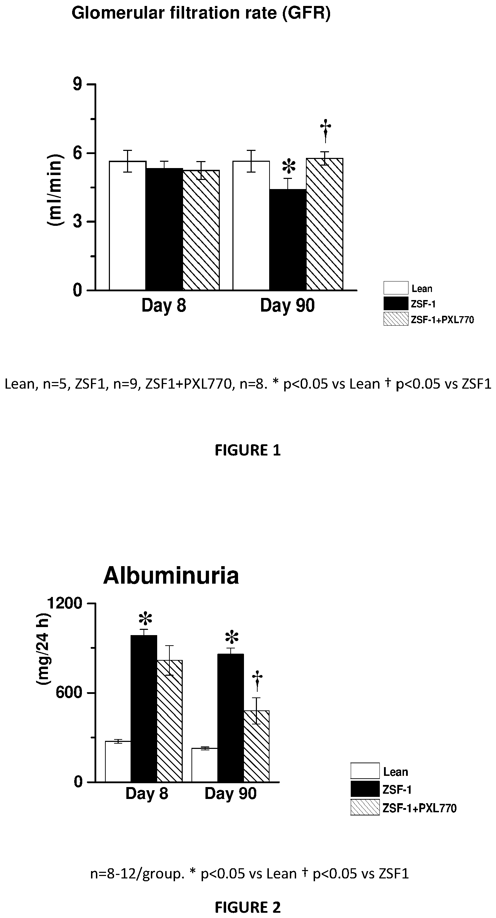

At 13 weeks of age, GFR was similar between Lean rats and untreated ZSF1 rats. In contrast, in 24-week old untreated ZSF1 rats, GFR was significantly reduced compared to age-matched Lean rats (Figure 1).

After 90 days of treatment, PXL770 significantly prevented the decrease in GFR observed in untreated ZSF1 rats. The kidney function (GFR) in PXL770-treated rats was normalized as it reached the same level of kidney function as in Lean rats (Figure 1).

### Albuminuria

At 13 weeks of age, untreated ZSF1 rats demonstrated a significant increase in urine albumin level compared to Lean rats (Figure 2). At 24 weeks of age, the significant increase in albumin level was still observed in untreated ZSF1 rats compared to age-matched Lean rats (Figure 2). After 8 days of PXL770 treatment, a decrease in albuminuria was observed in PXL770-treated ZSF1 rats compared to age-matched untreated ZSF1 rats.

After 90 days of PXL770 treatment, urine albumin level was significantly reduced in PXL770-treated ZSF1 rats compared to untreated ZSF1 rats (Figure 2).

### Urinary volume and glycosuria

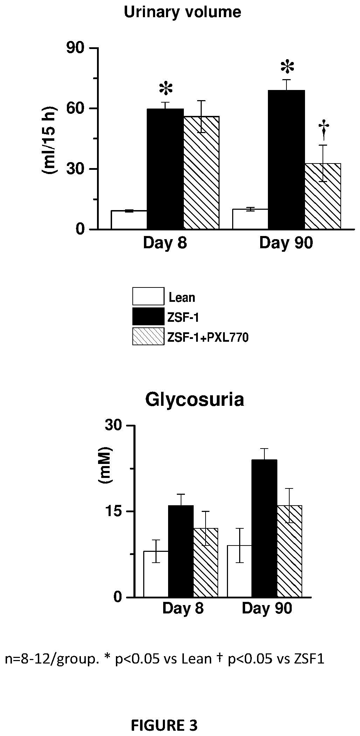

At 13 weeks of age, untreated ZSF1 rats demonstrated a significant increase in urine volume and a tendency to higher urine glucose level compared to Lean rats (Figure 3). At 24 weeks of age, the significant increase in urine volume in untreated ZSF1 rats was slightly more pronounced compared to 13 weeks of age, as well as the urine glucose level (Figure 3). 8 days of PXL770 treatment reduced glycosuria. In addition, 90 days of PXL770 treatment decreased significantly urine volume in PXL770-treated ZSF1 rats compared to untreated ZSF1 rats (Figure 3) and reduced glycosuria.

## Claims

1. A thienopyridone derivative of Formula (I): 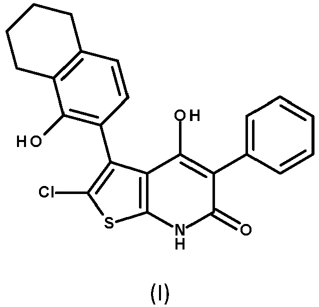
or a pharmaceutically acceptable salt and/or solvate thereof,
or a pharmaceutical composition comprising the same,
for use in the treatment of diabetic nephropathy.

2. The thienopyridone derivative of claim 1 or the pharmaceutical composition comprising the same for use according to claim 1, wherein said thienopyridone derivative is to be administered once or twice a day at a daily dose of 0.5mg to 3000 mg for a human patient, preferably from 20 mg to 1000 mg, more preferably from 60 mg to 500 mg.

3. The thienopyridone derivative of claim 1 or the pharmaceutical composition comprising the same for use according to claim 1 or 2, wherein said thienopyridone derivative or pharmaceutical composition is effective for preventing an increase in, or decreasing proteinuria, especially albuminuria, preventing or reducing kidney fibrosis, preventing the decrease or increasing glomerular filtration rate (GFR), reducing or preventing the increase in urine glucose level, reducing or preventing the increase in urine volume by the subject, and combinations thereof.

4. The thienopyridone derivative of claim 1 or the pharmaceutical composition comprising the same for use according to any one of claims 1 to 3, wherein said thienopyridone derivative or said pharmaceutical composition is to administered by oral route.

5. The thienopyridone derivative of claim 1 or a pharmaceutical composition comprising the same for use according to any one of claims 1 to 4, wherein said thienopyridone derivative is a monohydrate potassium salt of formula (Ia):

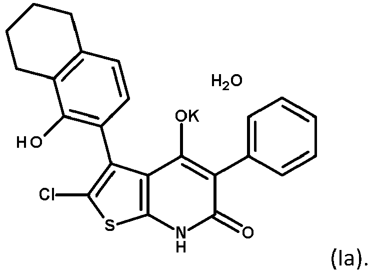

6. A thienopyridone derivative of Formula (I): 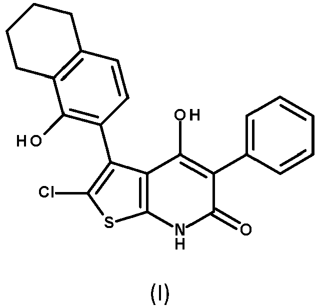 or a pharmaceutically acceptable salt and/or solvate thereof, for use in increasing or preventing the decrease of glomular filtration rate in a subject suffering from diabetic nephropathy.

7. The thienopyridone derivative of Formula (I) or a pharmaceutically acceptable salt and/or solvate thereof, for use according to claim 6, said thienopyridone derivative further decreases or prevents the increase of albumineria and/or reduces or prevents the increase in urine glucose level, and/or reduces or prevents the increase in urine volume in said subject.

8. The thienopyridone derivative of Formula (I) or a pharmaceutically acceptable salt and/or solvate thereof, for use according to claim 6 or 7 which is in the form of a monohydrate potassium salt of formula (Ia)

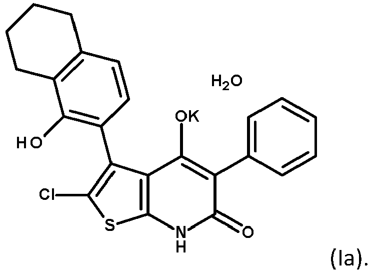

## Patentansprüche

1. Ein Thienopyridonderivat der Formel (I): 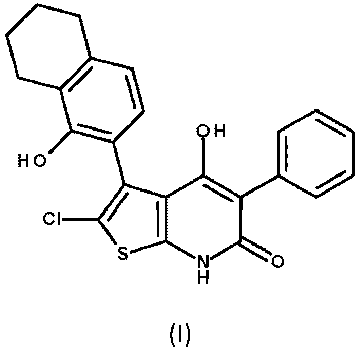
oder ein pharmazeutisch verträgliches Salz und/oder Solvat davon,
oder eine pharmazeutische Zusammensetzung, die dieses umfasst,
zur Verwendung bei der Behandlung von diabetischer Nephropathie.

2. Das Thienopyridonderivat nach Anspruch 1 oder die pharmazeutische Zusammensetzung, die dieses umfasst, zur Verwendung gemäß Anspruch 1, wobei das Thienopyridonderivat ein- oder zweimal am Tag mit einer Tagesdosis für einen menschlichen Patienten von 0,5 mg bis 3000 mg, vorzugsweise von 20 mg bis 1000 mg, noch bevorzugter von 60 mg bis 500 mg verabreicht wird.

3. Das Thienopyridonderivat nach Anspruch 1 oder die pharmazeutische Zusammensetzung, die dieses umfasst, zur Verwendung gemäß Anspruch 1 oder 2, wobei das Thienopyridonderivat oder die pharmazeutische Zusammensetzung wirksam einen Anstieg in Proteinurie verhindert oder Proteinurie verringert, insbesondere Albuminurie, Nierenfibrose verringert oder verhindert, den Abfall der glomerulären Filtrationsrate (GFR) verhindert oder diese anhebt, den Anstieg des Uringlukosespiegels verringert oder verhindert, das Urinvolumen in einem Patienten verringert oder dessen Anstieg verhindert, und Kombinationen davon.

4. Das Thienopyridonderivat nach Anspruch 1 oder die pharmazeutische Zusammensetzung, die dieses umfasst, zur Verwendung gemäß einem der Ansprüche 1 bis 3, wobei das Thienopyridonderivat oder die pharmazeutische Zusammensetzung auf oralem Wege verabreicht wird.

5. Das Thienopyridonderivat nach Anspruch 1 oder eine pharmazeutische Zusammensetzung, die dieses umfasst, zur Verwendung gemäß einem der Ansprüche 1 bis 4, wobei das Thienopyridonderivat ein Kaliumsalz Monohydrat der Formel (la) ist:

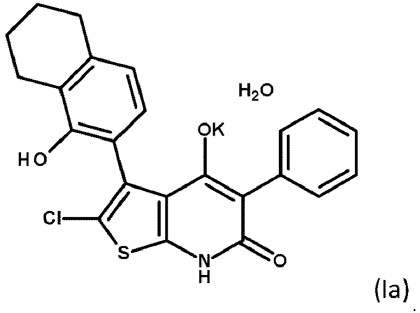

6. Ein Thienopyridonderivat der Formel (I): 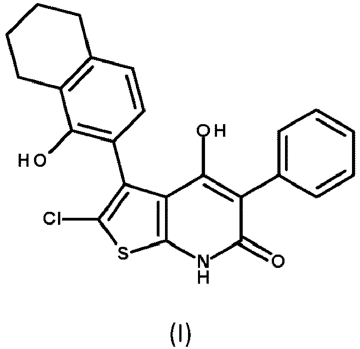 oder ein pharmazeutisch verträgliches Salz und/oder Solvat davon, zur Verwendung bei der Anhebung oder dem Verhinderung des Abfallens der glomerulären Filtrationsrate in einem Patienten, der an diabetischer Nephropathie leidet.

7. Das Thienopyridonderivat der Formel (I) oder ein pharmazeutisch verträgliches Salz und/oder Solvat davon zur Verwendung nach Anspruch 6, wobei das Thienopyridonderivat ferner Albuminurie verringert oder deren Anstieg verhindert und/oder den Anstieg des Uringlukosespiegels verringert oder verhindert, und/oder das Urinvolumen in diesem Patienten verringert oder dessen Zunahme verhindert.

8. Das Thienopyridonderivat der Formel (I) oder ein pharmazeutisch verträgliches Salz und/oder Solvat davon zur Verwendung nach Anspruch 6 oder 7, das in Form eines Kaliumsalz Monohydrats der Formel (la) 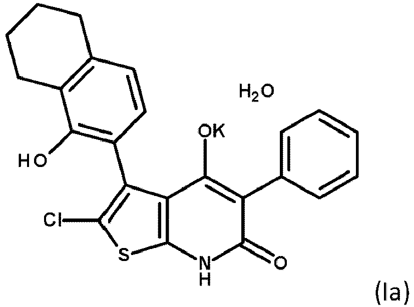 vorliegt.

## Revendications

1. Dérivé de thiénopyridone de formule (I) : 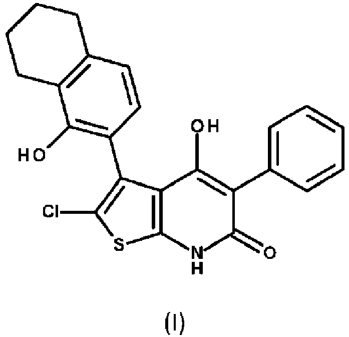
ou un solvate et/ou sel pharmaceutiquement acceptable de celui-ci,
ou une composition pharmaceutique le comprenant,
pour une utilisation dans le traitement d'une néphropathie diabétique.

2. Dérivé de thiénopyridone selon la revendication 1 ou composition pharmaceutique le comprenant pour une utilisation selon la revendication 1, dans lequel ledit dérivé de thiénopyridone est destiné à être administré une ou deux fois par jour à une dose quotidienne de 0,5 mg à 3 000 mg pour un patient humain, de préférence de 20 mg à 1 000 mg, mieux encore de 60 mg à 500 mg.

3. Dérivé de thiénopyridone selon la revendication 1 ou composition pharmaceutique le comprenant pour une utilisation selon la revendication 1 ou 2, dans lequel ledit dérivé de thiénopyridone ou composition pharmaceutique est efficace pour prévenir une augmentation d'une, ou diminuer une protéinurie, en particulier une albuminurie, prévenir ou réduire une fibrose rénale, prévenir une diminution du, ou augmenter le débit de filtration glomérulaire (GFR), réduire ou prévenir une augmentation du niveau urinaire de glucose, réduire ou prévenir une augmentation du volume urinaire chez le sujet, et les combinaisons de ceux-ci.

4. Dérivé de thiénopyridone selon la revendication 1 ou composition pharmaceutique le comprenant pour une utilisation selon l'une quelconque des revendications 1 à 3, dans lequel ledit dérivé de thiénopyridone ou la composition pharmaceutique est pour une administration par voie orale.

5. Dérivé de thiénopyridone selon la revendication 1 ou composition pharmaceutique le comprenant pour une utilisation selon l'une quelconque des revendications 1 à 4, dans lequel ledit dérivé de thiénopyridone est un sel potassique monohydraté de formule (Ia) :

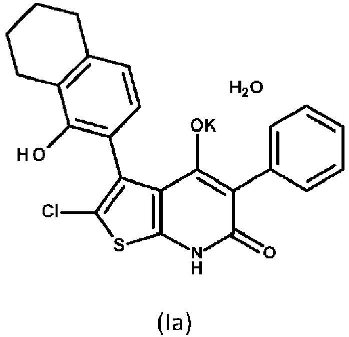

6. Dérivé de thiénopyridone de formule (I) : 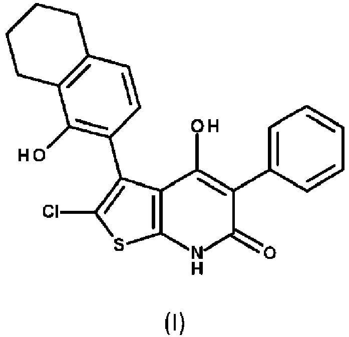 ou un solvate et/ou sel pharmaceutiquement acceptable de celui-ci, pour une utilisation dans l'augmentation ou la prévention de la diminution du débit de filtration glomérulaire chez un sujet souffrant d'une rétinopathie diabétique.

7. Dérivé de thiénopyridone de formule (I) ou un solvate et/ou sel pharmaceutiquement acceptable de celui-ci, pour une utilisation selon la revendication 6, dans lequel ledit dérivé de thiénopyridone en outre diminue ou prévient une augmentation d'une albuminémie et/ou réduit ou prévient une augmentation du niveau urinaire de glucose, et/ou réduit ou prévient une augmentation du volume urinaire chez ledit sujet.

8. Dérivé de thiénopyridone de formule (I) ou un solvate et/ou sel pharmaceutiquement acceptable de celui-ci, pour une utilisation selon la revendication 6 ou 7, qui est sous la forme d'un sel potassique monohydraté de formule (Ia) :
